## Europäisches Patentamt
## European Patent Office
## Office européen des brevets

(19)

(11) Veröffentlichungsnummer: **0 453 949 B1**

(12)
# EUROPÄISCHE PATENTSCHRIFT

(45) Veröffentlichungstag der Patentschrift: **07.09.94**

(51) Int. Cl.⁵: **C07C 39/17**, C07C 50/16, C01B 15/023

(21) Anmeldenummer: **91106173.7**

(22) Anmeldetag: **18.04.91**

(54) **Verfahren zur Herstellung von Alkyltetrahydroanthrahydrochinonlösungen und ihre Verwendung als Arbeitslösung bei der Wasserstoffperoxid-Herstellung nach dem Anthrachinonverfahren.**

(30) Priorität: **25.04.90 DE 4013090**

(43) Veröffentlichungstag der Anmeldung:
**30.10.91 Patentblatt 91/44**

(45) Bekanntmachung des Hinweises auf die
Patenterteilung:
**07.09.94 Patentblatt 94/36**

(84) Benannte Vertragsstaaten:
**AT BE CH DE ES FR GB IT LI NL SE**

(56) Entgegenhaltungen:
EP-A- 0 216 180  AT-B- 20 042
AT-B- 40 340  AT-B- 40 341
DE-A- 2 150 390  DE-A- 2 331 512
DE-A- 3 538 816  US-A- 2 495 229
US-A- 4 514 376

(73) Patentinhaber: **SOLVAY INTEROX GmbH**
**Dr.-Gustav-Adolph-Strasse 3**
**D-82049 Höllriegelskreuth (DE)**

(72) Erfinder: **Simon, Dietolf**
**Auf Dem Plänzer 3**
**W-5462 Bad Hönningen (DE)**
Erfinder: **Woost, Otmar**
**Wölper Strasse 35 A**
**W-3070 Nienburg (DE)**

(74) Vertreter: **Lauer, Dieter, Dr.**
**c/o Solvay Deutschland GmbH**
**Hans-Böckler-Allee 20**
**D-30002 Hannover (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf ein verbessertes Verfahren zur Herstellung von Alkyltetrahydro-anthrahydrochinonen in für die Wasserstoffperoxid-Herstellung geeigneten Lösungsmittelsystemen, wobei Lösungen von Alkyl-5,6,7,8-tetrahydro-anthrahydrochinonen erhalten werden, die direkt als Arbeitslösung für die Wasserstoffperoxid-Herstellung verwendet werden können oder aus denen gewünschtenfalls die Alkyltetrahydro-anthrahydrochinone oder nach deren Oxidation mit einem Sauerstoff-haltigen Gas auch die entsprechenden Alkyltetrahydro-anthrachinone isoliert werden können.

Bei der Herstellung von Wasserstoffperoxid nach dem Anthrachinonverfahren (AO-Verfahren) werden in hierfür geeigneten Lösungsmitteln gelöste Alkylanthrachinone als Reaktionsträger eingesetzt. Diese Lösungen der Alkylanthrachinone bezeichnet man als Arbeitslösung. Es ist auch bekannt, Alkylanthrachinone in Mischung mit hydrierstabilen Alkyl-5,6,7,8-tetrahydro-anthrachinonen in der Arbeitslösung für die Wasserstoffperoxid-Herstellung nach dem Anthrachinonverfahren zu verwenden. Die Tetrahydroanthrachinone entstehen dabei im Verfahrenszyklus in der Arbeitslösung sukzessiv von selbst als Hydriernebenprodukt der Alkylanthrachinone. Um die Vorteile der Alkyltetrahydro-anthrachinone bei der Wasserstoffperoxid-Herstellung besser nutzen zu können, werden daher im Stand der Technik die Tetrahydroderivate in separaten Synthesen gezielt hergestellt und isoliert, um sie direkt im AO-Verfahren einsetzen zu können. Ferner ist es aus der US 4 514 376 bekannt, den Ansteil an Tetrahydroderivaten gegenüber den Alkylanthrachinonen in der Arbeitslösung des AO-Verfahrens zu erhöhen, indem einer gewöhnlichen Alkylanthrachinon-Arbeitslösung eine durch Hydrierung (Palladium, 50 bis 400 kPa, Temperatur nicht größer als 50 °C) separat hergestellte Lösung mit erhöhtem Ansteil an Tetrahydroderivat maximal in einer solchen Menge zugesetzt wird, daß die Löslichkeit des Tetrahydroderivates in der Hydrierstufe des AO-Kreisprozesses nicht überschritten wird. Zwar ermöglicht das vorstehende Verfahren die Herstellung von Wasserstoffperoxid unter Verwendung von Arbeitslösungen mit verbessertem Verhältnis von Alkyltetrahydro-anthrahydrochinon zu Alkylanthrachinon. Nach dem Stand der Technik ist es bisher jedoch nicht möglich, in einem einfachen, direkten Verfahren im wesentlichen nur Alkyltetrahydro-anthrachinone als Reaktionsträger enthaltende Arbeitslösungen für die Wasserstoffperoxid-Herstellung nach dem AO-Verfahren herzustellen.

Aus der US-PS 2 495 229 ist ferner ein Verfahren zur Herstellung von Tetrahydroanthrachinonen bekannt, bei dem ein Anthrachinon in Lösungsmitteln wie insbesondere Dioxan, Tetralin oder Dekalin unter Rühren und gewöhnlichen bzw. niedrigen Drucken von 1 bis 3 Atmosphären (entspricht in etwa 101 bis 304 kPa) an einem porösen Nickel-Katalysator zunächst zum Tetrahydroanthrahydrochinonhydriert wird. Das in den Lösungsmitteln gebildete Tetrahydroanthrahydrochinon wird nachfolgend noch mittels durchperlender Luft zum Tetrahydroanthrachinon oxidiert. Diese Verfahrensweise ermöglicht es jedoch nicht, Alkyltetrahydro-anthrahydrochinone enthaltende Lösungen herzustellen, die direkt als Arbeitslösung für die Wasserstoffperoxid-Herstellung nach dem AO-Verfahren eingesetzt und in kurzer Zeit hierfür zur Verfügung gestellt werden können.

Aufgabe der Erfindung ist es daher, ein Verfahren zu schaffen, mit dem Alkylanthrachinone einerseits in hoher Ausbeute und Selektivität so zu den Tetrahydroderivaten hydriert werden können, daß die bei dieser Hydrierung anfallenden Alkyltetrahydro-anthrahydrochinon-Lösungen direkt als Arbeitslösung für die Wasserstoffperoxid-Herstellung nach dem AO-Verfahren verwendet werden können, und bei dem andererseits Nachteile wie Uneinheitlichkeit der Produktbildung weitestgehend vermieden werden.

Zur Lösung dieser Aufgabe schlägt die Erfindung ein Verfahren zur Herstellung von Alkyltetrahydro-anthrahydrochinon enthaltenden Lösungen (Produktlösung) durch heterogen katalysierte Druckhydrierung von Alkylanthrachinon enthaltenden Lösungen (Eduktlösungen) vor, welches sich dadurch auszeichnet, daß man eine Lösung von Alkylanthrachinon in einem für die Wasserstoffperoxid-Herstellung nach dem Anthrachinonverfahren geeigneten Lösungsmittelsystem in einem Hydrierreaktor vom Typ eines Schleifenreaktors mit Injektor-Mischdüse an einem Suspensionskatalysator oder Trägersuspensionskatalysator der Metalle Nickel, Platin oder Rhodium bei Temperaturen von 50 bis 70 °C und bei einem Druck von von 800 bis 1500 kPa hydriert, wobei man das Reaktionsgut kontinuierlich durch die Reaktionsschleife pumpt und wobei in der Injektor-Mischdüse unter intensiver Durchmischung mit dem Reaktionsgut Wasserstoffgas homogen zugemischt wird.

Dieses Verfahren ist sehr einfach durchzuführen und liefert mit hohen Ausbeuten und in hochselektiver Weise Lösungen der Tetrahydroderivate in der Hydrochinonform (Alkyltetrahydro-anthrahydrochinon), die in diesen Lösungen durch Oxidation mit einem sauerstoffhaltigen Gas in an sich bekannter Weise auch in die entsprechenden Alkyltetrahydro-anthrachinone überführbar sind. Die erfindungsgemäß hergestellten Alkyltetrahydro-anthrahydrochinon enthaltenden Lösungen lassen sich daher vorteilhaft als solche direkt im Anthrachinonverfahren zur Wasserstoffperoxidsynthese einsetzen, in dessen Verfahrensablauf sie dann zu Alkyltetrahydro-anthrachinon und $H_2O_2$ umgesetzt werden. Es versteht sich hierbei von selbst, daß die erfindungsgemäßen, Alkyltetrahydro-anthrahydrochinon enthaltenden Lösungen nicht nur ausschließlich für die Wasserstoffperoxid-Herstellung nach dem AO-Verfahren geeignet sind.

Außer der intensiven Durchmischung des Hydriergemisches sind für den Erfolg des erfindungsgemäßen Verfahrens die Hydrierparameter Temperatur und Druck von besonderer Bedeutung. Das erfindungsgemäße Verfahren wird bei einem Wasserstoffdruck von 800 bis 1500 kPa durchgeführt. Die Temperatur der Hydrierstufe liegt hierbei im Bereich zwischen 50 und 70 °C. Bei den genannten Drücken und Temperaturen ist das Verhältnis von Ausbeute, Selektivität und Geschwindigkeit der Umsetzung am günstigsten.

Bei der Durchführung des erfindungsgemäßen Verfahrens wird das zu hydrierende Alkylanthrachinon in einem Lösungsmittelsystem gelöst, welches an sich zur Herstellung von Arbeitslösungen für das AO-Verfahren geeignet ist. Insbesondere werden Lösungsmittelsysteme aus zwei oder mehr Lösungsmitteln verwendet, die für die unterschiedlichen Lösungseigenschaften von Chinonen und Hydrochinonen, wie sie im AO-Verfahren auftreten, gleichermaßen gut geeignet sind. Bevorzugt werden daher im erfindungsgemäßen Verfahren Gemische aus unpolaren, aromatischen Lösungsmitteln (Chinonlöser) und polaren Lösungsmitteln (Hydrochinonlöser) verwendet. Beispiele für verwendbare aromatische Lösungsmittel sind alkylsubstituierte Aromaten, insbesondere C9- und C10-Alkylbenzole bzw. deren Gemische. Beispiele für verwendbare polare Lösungsmittel sind höhere Alkohole (z.B. Diisobutylcarbinol, 2-Octanol), alkylierte und arylierte Harnstoffe, Phosphorsäureester (z.B. Trioctylphosphat), 2-Pyrrolidone, 2-Methylcyclohexylacetat oder deren Gemische. Beispiele für Lösungsmittelgemische sind Gemische aus C10-Alkylaromaten mit Diisobutylcarbinol, oder mit 2-Methylcyclohexylacetat.

Die Hydrierung erfolgt an einem heterogen vorliegenden Suspensions- oder Trägersuspensionskatalysator der Metalle Nickel, Platin oder Rhodium. In zweckmäßigen Ausgestaltungen des erfindungsgemäßen Verfahrens ist der Suspensionskatalysator Raney-Nickel oder die Trägersuspensionskatalysatoren sind Platin- oder Rhodiummetall auf Aluminiumoxid, Aluminiumsilikat, $SiO_2$, $TiO_2$ oder auf Kohlenstoff. Im Falle von Trägersuspensionskatalysatoren wird Platin- oder Rhodiummetallauf Aluminiumsilikat, $SiO_2$ oder Kohlenstoff bevorzugt. Als besonders bevorzugter Hydrierkatalysator wird Raney-Nickel verwendet, da es bei guter Selektivität die Edelmetall-Trägerkatalysatoren bezüglich der Kernhydriergeschwindigkeit übertrifft und darüber hinaus auch unter wirtschaftlichen Gesichtspunkten den anderen Katalysatoren weitaus überlegen ist.

Die Konzentration des Katalysators liegt im erfindungsgemäßen Verfahren bevorzugt in einem Bereich, der einer Suspensionsdichte des Suspensions- oder Trägersuspensionskatalysators von 0,2 bis 10 Gew.-% entspricht. Vorzugsweise beträgt die Suspensionsdichte 0,5 bis 2,5 Gew.-%.

Als Alkylanthrachinone, die hydriert werden, werden zweckmäßig die in Zweistellung alkylierten Anthrachinone verwendet. Besonders bevorzugt werden Anthrachinone hydriert, die in C2-Position mit einem C1- bis C10-Alkylrest, vorzugsweise mit einem C2- bis C5-Alkylrest substituiert sind. Zweckmäßige Alkylanthrachinone sind 2-Ethylanthrachinon, 2-Butylanthrachinone, 2-Amylanthrachinone oder deren Gemische. Besonders bevorzugt werden 2-Ethylanthrachinon, 2-tert.-Butylanthrachinon, 2-sek.-Amylanthrachinon, 2-tert.-Amylanthrachinon oder deren Gemische eingesetzt. In zweckmäßigen Ausgestaltungen des erfindungsgemäßen Verfahrens enthält die Eduktlösung das Alkylanthrachinon oder deren Gemische in einer Menge von 2 bis 20 Gew.-% (bezogen auf das Gesamtgewicht der Eduktlösung). Besonders bevorzugt sind hierbei Eduktlösungen, die das Alkylanthrachinon oder ein Gemisch aus Alkylanthrachinonen in einer Menge von 5 bis 15 Gew.-% enthalten.

Das erfindungsgemäße Verfahren bietet gegenüber der in situ-Bildung von Alkyltetrahydro-anthrahydrochinonen während der $H_2O_2$-Herstellung im AO-Verfahren den Vorteil, daß durch die AO-Prozeß-externe Kernhydrierung von Alkylanthrachinon eine separate Synthese von Alkyltetrahydroanthrahydrochinonen unter optimierten Bedingungen und zwar im Lösungsmittelsystem des AO-Verfahrens durchgeführt werden kann. Die Hydrierumsetzung ist innerhalb kurzer Zeiträume, d.h. maximal in etwa 2 bis 3 Stunden vollständig. In bevorzugten Varianten des erfindungsgemäßen Verfahrens kann die Hydrierung sogar in sehr kurzen Zeiträumen von etwa 20 Minuten bis 1 Stunde abgeschlossen werden. Das erfindungsgemäße Verfahren zeichnet sich ferner durch hohe Ausbeuten aus. Es sind sehr viele Hydrierzyklen möglich, ohne daß die Katalysatoraktivität deutlich nachläßt, insbesondere bei Verwendung von Raney-Nickel-Katalysator. Durch die hohe erreichte Selektivität wird die Bildung von Nebenprodukten die das Endprodukt (d.h. die in das AO-Verfahren zu überführende Alkyltetrahydro-anthrahydrochinon enthaltende Arbeitslösung) verunreinigen könnten, minimal gehalten. Die das Alkyltetrahydro-anthrahydrochinon enthaltende Produktlösung kann - sofern für die Herstellung der Produktlösung und für die Wasserstoffperoxid-Herstellung im AO-Verfahren derselbe Katalysator verwendet wird - direkt der Hydrierstufe des AO-Verfahrens zugeführt werden. Werden dagegen a) einerseits für die Herstellung der Produktlösung und im AO-Verfahren verschiedene Hydrierkatalysatoren eingesetzt und/oder soll b) andererseits die hergestellte Produktlösung der Oxidationsstufe des AO-Verfahrens zugeführt werden, ist die Abtrennung des Hydrierkatalysators aus der Produktlösung in jedem Fall erforderlich, um im Falle a) Probleme durch unerwünschte Vermischung verschiedener Katalysatoren und im Falle b) Probleme durch Zersetzung von in der Oxidationsstufe des AO-Verfahrens gebildetem Wasserstoffperoxid zu vermeiden. Die Abtrennung des Katalysators aus der Produktlösung wird durch an sich bekannte Maßnahmen bewerkstelligt, z.B. durch Dekantation, Filtration, Zentrifugation.

Eine Isolierung der Alkyltetrahydro-anthrahydrochinone aus der Produktlösung ist für deren Verwendung bei der Wasserstoffperoxid-Herstellung nach dem AO-Verfahren nicht erforderlich. Das erfindungsgemäß gebildete Alkyltetrahydro-anthrahydrochinon liegt vorteilhaft in solcher Konzentration und Reinheit in einem für die $H_2O_2$-Synthese nach dem AO-Verfahren geeigneten Lösungsmittelsystem vor, daß die nach dem oben beschriebenen Verfahren hergestellten Lösungen direkt, d.h. ohne weitere Reinigung oder Aufkonzentrierung, in das AO-Verfahren zur $H_2O_2$-Herstellung überführt werden können.

Die Erfindung betrifft daher weiterhin ein Verfahren zur Herstellung von Wasserstoffperoxid nach dem Anthrachinon-Verfahren, bei dem in an sich bekannter Weise eine Arbeitslösung zyklisch durch einen Arbeitskreislauf, umfassend eine Hydrierstufe, eine Oxidationsstufe und eine Extraktionsstufe, geführt wird, dadurch gekennzeichnet, daß man eine im wesentlichen nur Alkyltetrahydro-anthrahydrochinon als Reaktionsträger enthaltende Arbeitslösung verwendet, die zunächst in einer vom Arbeitskreislauf getrennten Hydrierstufe durch heterogen katalysierte Druckhydrierung einer Alkylanthrachinon enthaltenden Lösung hergestellt wird, indem man eine Lösung von Alkylanthrachinon in einem für die Wasserstoffperoxid-Herstellung nach dem Anthrachinonverfahren geeigneten Lösungsmittelsystem in einem Hydrierreaktor vom Typ eines Schleifenreaktors mit Injektor-Mischdüse an einem Suspensionskatalysator oder Trägersuspensionskatalysator der Metalle Nickel, Platin oder Rhodium bei Temperaturen von 50 bis 70 °C und bei einem Druck von 800 bis 1500 kPa hydriert, wobei man das Reaktionsgut kontinuierlich durch die Reaktionsschleife pumpt und wobei in der Injektor-Mischdüse unter intensiver Durchmischung mit dem Reaktionsgut Wasserstoffgas homogen zugemischt wird, und daß danach diese Arbeits Lösung gegebenenfalls nach Abtrennung des Suspensions- oder Trägersuspensionskatalysators, dem Arbeitskreislauf (unter Beachtung der weiter oben beschriebenen Maßgaben) zugeführt wird.

Die Vorteile dieses Verfahrens zur $H_2O_2$-Herstellung nach dem AO-Verfahren ergeben sich direkt aus den Vorteilen der eingesetzten Alkyltetrahydro-anthrahydrochinon-Lösung, die nach dem weiter oben beschriebenen erfindungsgemäßen Verfahren zur Herstellung von Alkyltetrahydroanthrahydrochinon enthaltenden Lösungen gewonnen wurden. Die hohe Ausbeute, Selektivität und Reinheit sowie die nur kurzen Hydrierzeiten bei der erfindungsgemäßen Herstellung der Alkyltetrahydro-anthrahydrochinon-Lösungen in einem für das AO-Verfahren geeigneten Lösungsmittelgemisch erlauben ein hohes Maß an Flexibilität bei der $H_2O_2$-Herstellung nach dem AO-Verfahren, wodurch nicht zuletzt auch kurzfristige Produktivitätssteigerungen ermöglicht werden.

Die Alkyltetrahydro-anthrahydrochinone können darüber hinaus nach Abtrennung des Katalysators aus den erfindungsgemäß hergestellten Produktlösungen - insbesondere für andere Anwendungsbereiche als die $H_2O_2$-Herstellung nach dem AO-Verfahren - auch aus den erfindungsgemäß hergestellten Produktlösungen isoliert werden. Falls hierbei nicht die Alkyltetrahydro-anthrahydrochinone selbst (also die Hydrochinonform des Produktes), sondern die Chinonform (Alkyltetrahydro-anthrachinon) gewünscht wird, kann nach zuvor erfolgter Abtrennung des Katalysators die in der Produktlösung vorliegende Hydrochinonform durch Umsetzung mit einem Sauerstoff-haltigen Gas (z.B. Luft) auch zur Chinonform oxidiert und anschließend das dabei gebildete Alkyltetrahydro-anthrachinon - gegebenenfalls nach Abtrennung des gebildeten Wasserstoffperoxids - isoliert werden. Die Isolierung von Alkyltetrahydro-anthrahydrochinon oder Alkyltetrahydro-anthrachinon kann beispielsweise auch aus Transportgründen für die Chinonzulieferer von $H_2O_2$-Anlagen interessant sein. Die Erfindung ermöglicht daher auch ein Verfahren zur Herstellung von Alkyltetrahydro-anthrahydrochinon oder Alkyltetrahydro-anthrachinon, welches sich dadurch auszeichnet, daß man gemäß dem vorstehend beschriebenen erfindungsgemäßen Verfahren zunächst durch heterogen katalysierte Druckhydrierung von Alkylanthrachinon enthaltenden Lösungen (Eduktlösung) und anschließende Abtrennung des Katalysators eine katalysatorfreie, Alkyltetrahydro-anthrahydrochinon enthaltende Lösung (katalysatorfreie Produktlösung) herstellt und daß man anschließend entweder a) aus der katalysatorfreien Produktlösung das Alkyltetrahydro-anthrahydrochinon isoliert oder daß man b) das in der katalysatorfreien Produktlösung enthaltene Alkyltetrahydro-anthrahydrochinon durch Umsetzung mit einem Sauerstoff-haltigen Gas oxidiert und dann das gebildete Alkyltetrahydro-anthrachinon isoliert. Die Isolierung der Produkte wird hierbei durch an sich bekannte Maßnahmen bewerkstelligt, z.B. durch Abkühlung der die Produkte enthaltenden Lösung und anschließende Filtration bzw. Zentrifugation des gebildeten Niederschlages; andererseits können die Produkte auch durch Abdestillieren des Lösungsmittels gewonnen werden. Die festen Produkte fallen hierbei auf Grund des hohen Hydrierumsatzes und der guten Selektivität der Hydrierung mit hoher Ausbeute bei gleichzeitig hoher Reinheit an.

Beispiele

Die nachfolgenden Beispiele wurden in einem Hydrierreaktor vom Typ eines Schleifenreaktors durchgeführt, dessen Aufbau und Funktionsprinzip z.B. in der europäischen Patentanmeldung 70 797 näher beschrieben sind und der die für das Verfahren der Erfindung u.a. gewünschte intensive Durchmischung des Hydrieransatzes aufgrund seiner hohen Mischungseffizienz besonders gut gewährleistete.

Das Reaktionsgut (Lösung von Alkylanthrachinonen und Katalysator), dessen jeweilige Zusammensetzung der Tabelle 1 zu entnehmen ist, wurde zunächst in einem Behälter vorgelegt und das heterogene Gemisch anschließend in die Reaktionsschleife des Reaktors überführt. Wesentliche Teile der Reaktionsschleife waren: ein als eigentlicher Reaktionsbehälter dienender Autoklav mit einer von der Deckenwand des Reaktionsbehälters von oben her in diesen einmündenden Injektor-Mischdüse und mit einer Öffnung im Boden des Reaktionsbehälters, sowie weiterer Zu- und Ableitungen für das Reaktionsgut bzw. die Produktlösung; eine Umwälzpumpe; ein Wärmetauscher; eine Gaszufuhrleitung zur Injektor-Mischdüse; und eine Schleifenleitung. Die Schleifenleitung verband die genannten Teile des Reaktors in der Reihenfolge Boden des Reaktionsbehälters, Umwälzpumpe, Wärmetauscher, Injektor-Mischdüse zur sogenannten Reaktionsschleife.

Während der Hydrierung wurde das Reaktionsgut kontinuierlich durch die Reaktionsschleife gepumpt, wobei in der Injektor-Mischdüse Wasserstoff nach dem Injektorprinzip homogen zugemischt wurde. In der Injektor-Mischdüse wurden das Hydriergas und das Reaktionsgut intensiv durchmischt und durch die in der Düse erzeugten hohen Scherkräfte die Hydrierung unter den erfindungsgemäßen Bedingungen vorteilhaft unterstützt, so daß die Umsetzung der Alkylanthrachinone zu Alkyltetrahydro-anthrahydrochinonen mit hoher Ausbeute und Selektivität verlief und auch eine wesentliche Erhöhung der Hydriergeschwindigkeit erzielt wurde.

Die Hydrierungen von jeweils 45 kg Reaktionsgut erfolgten unter Variation von Katalysator, Alkylanthrachinon, Alkylanthrachinon-Konzentration, Temperatur, Druck, Lösungsmittel und Reaktionsdauer. Tabelle 1 gibt einen Überblick über die erhaltenen Ergebnisse.

Zum Beispiel 2 ist ergänzend anzumerken, daß der dort eingesetzte Raney-Nickel-Katalysator in 6 Folgeansätzen wiederverwendet werden konnte, ohne daß im Verlauf der Folgehydrierungen sich eine signifikante Aktivitätsabnahme des Katalysators einstellte. Die in den Umsetzungen der vorstehenden Beispiele erhaltenen Lösungen konnten nach Abtrennung des suspendierten Katalysators (im Falle des Einsatzes gleicher Katalysatoren für die Herstellung der Alkyltetrahydro-anthrahydrochinon enthaltenden Produktlösungen einerseits und im AO-Verfahren zur $H_2O_2$-Herstellung andererseits, gegebenenfalls auch ohne Abtrennung des Katalysators) einem Produktionskreislauf zur Herstellung von Wasserstoffperoxid nach dem Anthrachinon-Verfahren zugeführt werden. Die Herstellung von Wasserstoffperoxid unter Verwendung der nach dem erfindungsgemäßen Verfahren hergestellten Alkyltetrahydro-anthrahydrochinon enthaltenden Lösungen konnte hinsichtlich der Hydrierstabilität des Reaktionsträgers Alkyltetrahydro-anthrahydrochinon/-anthrachinon in besonders vorteilhafter Weise durchgeführt werden; d.h. die Bildung unerwünschter Hydriernebenprodukte in der AO-Hydrierstufe wurde weitgehend vermieden, wodurch der Verbrauch an Chinon vermindert und Chinon eingespart wurde.

Tabelle 1

| Bei-spiel Nr. | Eduktlösung (Konzentration in g/kg Lösung) Alkyl-rest/Anthra-chinon* | Lösemittelgemisch | Katalysator (% Suspen-sions-dichte) | Tempe-ratur °C | Druck kPa | Reaktions-dauer min. | Hydrier-Umsatz*** % | Selektivität der Tetra-hydroderi-vat-Bildung**** % |
|---|---|---|---|---|---|---|---|---|
| 1 | 2-Amyl (100) | Diisobutylcarbinol (270)/C10-Alkyl-aromatengemisch (630) | Raney-Nickel(1,0) | 50 | 800 | 110 | 96,1 | 94,5 |
| 2 | wie 1 | wie 1 | wie 1 | 70 | 1500 | 22 | 96,4 | 95,0 |
| 3 | wie 1 | wie 1 | wie 1 | 70 | 800 | 24 | 92,2 | 93,2 |
| 4 | wie 1 | wie 1 | wie 1 | 50 | 1500 | 50 | 93,5 | 97,0 |
| 5 | 2-Amyl (70) 2-Ethyl (30)** | wie 1 | wie 1 | 70 | 1500 | 17 | 93,0 | 96,3 |
| 6 | 2-Ethyl (50) | 2-Methylcyclo-hexylacetat (390)/C-10 Alkylaroma-tengemisch (560) | wie 1 | 70 | 1500 | 27 | 93,7 | 97,9 |
| 7 | 2-Amyl (100) | Diisobutylcarbinol (300)/C-10 Alkyl-aromatengemisch (600) | 2 % Pt auf Aluminium-silikatträ-ger (0,8) | 70 | 800 | 170 | 84,0 | 93,9 |
| 8 | wie 7 | wie 7 | wie Bei-spiel 7, jedoch mit 2,2 % Susp. dichte | 70 | 1200 | 30 | 99,3 | 99 |

\* 2-Amylanthrachinon = Isomerengemisch aus sek. und tert. Amylanthrachinon
\*\* Gemisch aus 2-Amyl- und 2-Ethylanthrachinon
\*\*\* bezogen auf eingesetztes Alkylanthrachinon
\*\*\*\* gebildete Menge an Tetrahydroderivat bezogen auf eingesetzte Menge an Alkylanthrachinon

## Patentansprüche

1. Verfahren zur Herstellung von Alkyltetrahydroanthrahydrochinon enthaltenden Lösungen (Produktlösung) durch heterogen katalysierte Druckhydrierung von Alkylanthrachinon enthaltenden Lösungen (Eduktlösung), dadurch gekennzeichnet, daß man eine Lösung von Alkylanthrachinon in einem für die Wasserstoffperoxid-Herstellung nach dem Anthrachinonverfahren geeigneten Lösungsmittelsystem in einem Hydrierreaktor vom Typ eines Schleifenreaktors mit Injektor-Mischdüse an einem Suspensionskatalysator oder Trägersuspensionskatalysator der Metalle Nickel, Platin oder Rhodium bei Temperaturen von 50 bis 70°C und bei einem Druck von 800 bis 1500 kPa hydriert, wobei man das Reaktionsgut kontinuierlich durch die Reaktionsschleife pumpt und wobei in der Injektor-Mischdüse unter intensiver Durchmischung mit dem Reaktionsgut Wasserstoffgas homogen zugemischt wird.

2. Verfahren nach Anspruch 1, dadurch gekennzeichnet, daß darin als Lösungsmittelsystem ein Gemisch aus unpolaren, aromatischen Chinonlösern und polaren Hydrochinonlösern verwendet wird.

3. Verfahren nach Anspruch 2, dadurch gekennzeichnet, daß als Lösungsmittelsystem ein Gemisch aus C10-Alkylaromaten mit Diisobutylcarbinol oder mit 2-Methylcyclohexylacetat verwendet wird.

4. Verfahren nach einem der vorhergehenden Ansprüche, dadurch gekennzeichnet, daß der Suspensionskatalysator Raney-Nickel ist oder der Trägersuspensionskatalysator Platin- oder Rhodiummetall auf Aluminiumoxid, Aluminiumsilikat, $SiO_2$, $TiO_2$ oder auf Kohlenstoff, vorzugsweise auf Aluminiumsilikat, $SiO_2$ oder Kohlenstoff, ist.

5. Verfahren nach einem der vorhergehenden Ansprüche dadurch gekennzeichnet, daß die Suspensionsdichte des Suspensions- oder Trägersuspensionskatalysators 0,2 bis 10 Gew.-%, vorzugsweise 0,5 bis 2,5 Gew.-%, beträgt.

6. Verfahren nach einem der vorhergehenden Ansprüche, dadurch gekennzeichnet, daß das Alkylanthrachinon ein in C2-Position mit einem C1- bis C10-Alkylrest, vorzugsweise mit einem C2- bis C5-Alkylrest, substituiertes Alkylanthrachinon ist.

7. Verfahren nach einem der vorhergehenden Ansprüche, dadurch gekennzeichnet, daß das Alkylanthrachinon 2-Ethylanthrachinon, ein 2-Butylanthrachinon oder ein 2-Amylanthrachinon oder ein Gemisch derselben, vorzugsweise 2-Ethylanthrachinon, 2-tert.-Butylanthrachinon, 2-sek.-Amylanthrachinon, 2-tert.-Amylanthrachinon oder ein Gemisch derselben, ist.

8. Verfahren nach einem der vorhergehenden Ansprüche, dadurch gekennzeichnet, daß die Eduktlösung das Alkylanthrachinon oder ein Gemisch aus Alkylanthrachinonen in einer Menge von 2 bis 20 Gew.-%, vorzugsweise 5 bis 15 Gew.-%, enthält (bezogen auf das Gesamtgewicht der Eduktlösung).

9. Verfahren zur Herstellung von Wasserstoffperoxid nach dem Anthrachinon-Verfahren, bei dem in an sich bekannter Weise eine Arbeitslösung zyklisch durch einen Arbeitskreislauf, umfassend eine Hydrierstufe, eine Oxidationsstufe und eine Extraktionsstufe, geführt wird, dadurch gekennzeichnet, daß man eine im wesentlichen nur Alkyltetrahydro-anthrahydrochinon als Reaktionsträger enthaltende Arbeitslösung verwendet, die zunächst in einer vom Arbeitskreislauf getrennten Hydrierstufe durch heterogen katalysierte Druckhydrierung einer Alkylanthrachinon enthaltenden Lösung hergestellt wird, indem man eine Lösung von Alkylanthrachinon in einem für die Wasserstoffperoxid-Herstellung nach dem Anthrachinonverfahren geeigneten Lösungsmittelsystem in einem Hydrierreaktor vom Typ eines Schleifenreaktors mit Injektor-Mischdüse an einem Suspensionskatalysator oder Trägersuspensionskatalysator der Metalle Nickel, Platin oder Rhodium bei Temperaturen von 50 bis 70°C und bei einem Druck von 800 bis 1500 kPa hydriert, wobei man das Reaktionsgut kontinuierlich durch die Reaktionsschleife pumpt und wobei in der Injektor-Mischdüse unter intensiver Durchmischung mit dem Reaktionsgut Wasserstoffgas homogen zugemischt wird, und daß danach diese Arbeitslösung, gegebenenfalls nach Abtrennung des Suspensions- oder Trägersuspensionskatalysator, dem Arbeitskreislauf zugeführt wird.

10. Verfahren nach Anspruch 9, dadurch gekennzeichnet, daß darin als Lösungsmittelsystem ein Gemisch aus unpolaren, aromatischen Chinonlösern und polaren Hydrochinonlösern, vorzugsweise ein Gemisch aus C10-Alkylaromaten mit Diisobutylcarbinol oder mit 2-Methylcyclohexylacetat, verwendet wird.

## Claims

1. Process for the production of solutions containing alkyltetrahydroanthrahydroquinone (product solution) by the heterogeneously catalysed pressure hydrogenation of solutions containing alkylanthraquinone (starting solution), characterised in that a solution of alkylanthraquinone in a solvent system suitable for the production of hydrogen peroxide according to the anthraquinone process is hydrogenated in a hydrogenation reactor of the loop reactor type with an injector mixing nozzle on a suspension catalyst or carrier suspension catalyst of the metals nickel, platinum or rhodium at temperatures of 50 to 70°C and a pressure of 800 to 1500 kPa, in which the reaction mixture is pumped continuously through the reaction loop and in which hydrogen gas is admixed with the reaction mixture homogeneously and with intensive intermixing in the injector nozzle.

2. Process according to claim 1, characterised in that a mixture of non-polar, aromatic quinone solvents and polar hydroquinone solvents is used therein as the solvent system.

3. Process according to claim 2, characterised in that a mixture of C10-alkyl aromatics with diisobutyl carbinol or with 2-methyl cyclohexyl acetate is used as the solvent system.

4. Process according to one of the preceding claims, characterised in that the suspension catalyst is Raney nickel or the carrier suspension catalyst is platinum or rhodium metal on aluminium oxide, aluminium silicate, $SiO_2$, $TiO_2$ or on carbon, preferably on aluminium silicate, $SiO_2$ or carbon.

5. Process according to one of the preceding claims, characterised in that the suspension density of the suspension or carrier suspension catalyst is 0.2 to 10% by weight, preferably 0.5 to 2.5% by weight.

6. Process according to one of the preceding claims, characterised in that the alkylanthraquinone is an alkylanthraquinone substituted in the C2 position with a C1 to C10 alkyl radical, preferably with a C2 to C5 alkyl radical.

7. Process according to one of the preceding claims, characterised in that the alkylanthraquinone is 2-ethylanthraquinone, a 2-butylanthraquinone or a 2-amylanthraquinone or a mixture thereof, preferably 2-ethylanthraquinone, 2-tert. butylanthraquinone, 2-sec. amylanthraquinone, 2-tert. amylanthraquinone or a mixture thereof.

8. Process according to one of the preceding claims, characterised in that the starting solution contains the alkylanthraquinone or a mixture of alkylanthraquinone in a quantity of 2 to 20% by weight, preferably of 5 to 15% by weight (based on the total weight of the starting solution).

9. Process for the production of hydrogen peroxide according to the anthraquinone process during which a working solution is passed cyclically through an operating cycle in the known way, which cycle comprises a hydrogenation stage, an oxidation stage and an extraction stage, characterised in that a working solution is used which essentially contains only alkyltetrahydroanthrahyroquinone as reaction carrier, which working solution is initially produced in a hydrogenation stage separate from the operating cycle by the heterogeneously catalysed pressure hydrogenation of a solution containing alkylanthraquinone, by hydrogenating a solution of alkylanthraquinone in a solvent system suitable for the production of hydrogen peroxide according to the anthraquinone process in a hydrogenation reactor of the loop reactor type with injector mixing nozzle on a suspension catalyst or carrier suspension catalyst of the metals nickel, platinum or rhodium at temperatures of 50 to 70°C and a pressure of 800 to 1500 kPa, the reaction mixture being pumped continuously through the reaction loop and hydrogen gas being homogeneously admixed in the injector mixing nozzle with intensive mixing with the reaction mixture, and that this working solution is subsequently reconducted to the operating cycle, if necessary after separating off the suspension or carrier suspension catalyst.

10. Process according to claim 9, characterised in that a mixture of non-polar, aromatic quinone solvents and polar hydroquinone solvents, preferably a mixture of C10 alkyl aromatics with diisobutyl carbinol or with 2-methyl cyclohexyl acetate is used as the solvent systems.

**Revendications**

1. Procédé pour la préparation de solutions contenant de l'alkyltétrahydroanthrahydroquinone (solution de produit) par hydrogénation sous pression à catalyse hétérogène de solutions contenant de l'alkylanthraquinone (solution d'éduit), caractérisé en ce qu'on soumet à une hydrogénation une solution d'alkylanthraquinone, dans un système de solvants approprié pour la préparation de peroxyde d'hydrogène conformément au procédé à l'anthraquinone, dans un réacteur d'hydrogénation du type réacteur à boucle muni d'une tuyère de mélange à injecteur sur un catalyseur en suspension ou supporté en suspension d'un métal de nickel, de platine ou de rhodium, à des températures de 50 à 70 °C et sous une pression de 800 à 1500 kPa, dans lequel on pompe en continu la matière réactionnelle à travers la boucle réactionnelle et dans lequel, dans la tuyère de mélange à injecteur, en mélangeant de manière intensive, on mélange à la matière réactionnelle du gaz d'hydrogène jusqu'à homogénéité.

2. Procédé selon la revendication 1, caractérisé en ce qu'on y utilise, comme système de solvants, un mélange de solvants apolaires aromatiques de quinone et de solvants polaires d'hydroquinone.

3. Procédé selon la revendication 2, caractérisé en ce qu'on utilise, comme système de solvants, un mélange constitué par des composés alkyl(en C10)aromatiques avec le diisobutylcarbinol ou avec l'acétate de 2- méthylcyclohexyle.

4. Procédé selon l'une quelconque des revendications précédentes, caractérisé en ce que le catalyseur en suspension est le nickel de Raney, ou bien le catalyseur supporté en suspension est un catalyseur métallique de platine ou de rhodium sur un support d'oxyde d'aluminium, sur du silicate d'aluminium, sur $SiO_2$, sur $TiO_2$ ou encore sur du carbone, de préférence sur du silicate d'aluminium, sur $SiO_2$ ou sur du carbone.

5. Procédé selon l'une quelconque des revendications précédentes, caractérisé en ce que la densité de la suspension du catalyseur en suspension ou du catalyseur supporté en suspension s'élève de 0,2 à 10 % en poids, de préférence de 0,5 à 2,5 % en poids.

6. Procédé selon l'une quelconque des revendications précédentes, caractérisé en ce que l'alkylanthraquinone est une alkylanthraquinone substituée en position C2 par un radical alkyle en C1 à C10, de préférence par un radical alkyle en C2 à C5.

7. Procédé selon l'une quelconque des revendications précédentes, caractérisé en ce que l'alkylanthraquinone est la 2-éthylanthraquinone, une 2-butylanthraquinone ou une 2-amylanthraquinone, ou encore un mélange de ces dernières, de préférence la 2-éthylanthraquinone, la 2-tert.-butylanthraquinone, la 2-sec.-amylanthraquinone, la 2-tert.-amylanthraquinone ou encore un mélange de ces dernières.

8. Procédé selon l'une quelconque des revendications précédentes, caractérisé en ce que la solution d'éduit contient l'alkylanthraquinone ou un mélange d'alkylanthraquinones en une quantité de 2 à 20 % en poids, de préférence de 5 à 15 % en poids (rapportée au poids total de la solution d'éduit).

9. Procédé pour la préparation de peroxyde d'hydrogène conformément au procédé à l'anthraquinone, dans lequel, d'une manière connue en soi, on guide une solution de travail de manière cyclique à travers un circuit de travail comprenant une étape d'hydrogénation, une étape d'oxydation et une étape d'extraction, caractérisé en ce qu'on utilise une solution de travail contenant, comme support réactionnel, essentiellement uniquement une alkyltétrahydro-anthrahydroquinone, quel'on prépare d'abord, dans une étape d'hydrogénation séparée du circuit de travail, par hydrogénation sous pression à catalyse hétérogène d'une solution contenant de l'alkylanthraquinone, dans laquelle on soumet à une hydrogénation une solution d'alkylanthraquinone dans un système de solvants approprié conformément au procédé à l'anthraquinone pour la préparation de peroxyde d'hydrogène dans un réacteur d'hydrogénation du type d'un réacteur à boucle muni d'une tuyère de mélange à injecteur sur un catalyseur en suspension ou un catalyseur supporté en suspension d'un métal de nickel, de platine ou de rhodium, à des températures de 50 à 70 °C et sous une pression de 800 à 1500 kPa, dans lequel on pompe en continu la matière réactionnelle à travers la boucle réactionnelle et dans lequel, dans la tuyère de mélange à injecteur, en mélangeant de manière intensive, on mélange à la matière réactionnelle du gaz d'hydrogène jusqu'à homogénéité, et en ce que, par la suite, on achemine cette solution de travail au circuit de travail, éventuellement après séparation du catalyseur en suspension ou du catalyseur supporté en suspension.

10. Procédé selon la revendication 9, caractérisé en ce qu'on y utilise, comme système de solvants, un mélange de solvants aromatiques apolaires de quinone et de solvants polaires d'hydroquinone, de préférence un mélange constitué par des composés alkyl(en C10)aromatiques avec le diisobutylcarbinol ou avec l'acétate de 2-méthylcyclohexyle.